# EUROPEAN PATENT SPECIFICATION

(11) **EP 2 870 887 B1**
(45) Date of publication and mention of the grant of the patent: **28.06.2017**
(21) Application number: 12880826.8
(22) Date of filing: 09.07.2012
(51) Int. Cl.: A24F 47/00

(54) **ELECTRONIC CIGARETTE**
ELEKTRONISCHE ZIGARETTE
CIGARETTE ÉLECTRONIQUE

(43) Date of publication of application: 13.05.2015
(73) Proprietor: Huizhou Kimree Technology Co., Ltd. Shenzhen Branch, Shenzhen City, Guangdong 518040 (CN)
(72) Inventor: LIU, Qiuming, Huizhou, Guangdong 516000 (CN)
(74) Representative: Beetz & Partner mbB
(86) International application number: PCT/CN2012/078360
(87) International publication number: WO 2014/008623

(56) References cited:
- CN-U- 201 733 855
- CN-U- 202 112 305
- CN-Y- 2 591 886
- CN-Y- 200 997 909
- CN-Y- 201 408 820
- KR-A- 20080 030 247
- KR-A- 20120 057 459
- US-A1- 2006 131 431
- US-A1- 2007 126 290

## Description

### BACKGROUND OF THE INVENTION

### 1. FIELD OF THE INVENTION

The present invention relates to an electronic cigarette, and particularly to an electronic cigarette internally equipped with a universal serial bus (USB) connector.

### 2. RELATED ART

A conventional electronic cigarette includes a cigarette rod in which a control circuit unit is installed, and an universal serial bus (USB) connector is disposed on an outside portion of the cigarette rod and connected to the control circuit unit through a conductor. The conventional cigarette rod generally has an inhaling rod and a battery rod, wherein the inhaling rod and the battery rod are screwingly connected.

The conventional electronic cigarette has drawbacks as follows: first, it is inconvenient to use and slightly, because the USB connector is disposed at the outside portion of the cigarette rod; second, it is inconvenient and time consuming to disassemble the inhaling rod with the battery rod by screw thread.

Document CN-U-201733855 discloses an electronic cigarette according to the preamble of independent claim 1.

### SUMMARY OF THE INVENTION

An object of the present invention is to provide an electronic cigarette having a USB connector and a conductor both disposed in the electronic cigarette, whereby providing a convenient use, easy and quick assembly, and an aesthetic appearance.

To achieve the above object, an electronic cigarette of the present invention, comprises: a cigarette body and a connecting device. The connecting device is retractably disposed in the cigarette body so as to be hidden therein, and comprises a connector and a conductor of which a length is extendable, the conductor capable of being retractably received in the cigarette body, one end of the conductor connected to the connector, the other end of the conductor electrically connected to a circuit control unit disposed in the cigarette body. The connector is capable of being retractably disposed in the cigarette body and extends out of the cigarette body through the retractable conductor to a pre-determined distance. When the connecting device is not in use, the conductor is foldably retracted to be hidden within the cigarette body together with the connector, while the connecting device is in use, the connecting device is pulled away and the conductor extends to a predetermined length, whereby the connector electrically connects the electronic cigarette to an external electronic device or power.

According to another embodiment of the present invention, the connecting device further comprises a support base disposed in the cigarette body for accommodating and positioning the connector, a first accommodating chamber is defined in the support base for accommodating the connector and the conductor, the first accommodating chamber having a wall body defining a cylindrical front portion for allowing the connector and the conductor to extend out of or retract to the support base, and the cylindrical front portion corresponds to an inner wall of the cigarette body.

According to another embodiment of the present invention, the conductor comprises a movable conductor and a fixing conductor both integrally connected with each other over a coupling element disposed in between the movable conductor and the fixing conductor, the coupling element is mounted to the support base, one end portion of the movable conductor is electrically connected to one end portion of the fixing conductor through the coupling element, the conductor is electrically connected to the connector through the one end portion of the movable conductor, and the conductor is electrically connected to a control circuit unit in the electronic cigarette through another end portion of the fixing conductor.

According to another embodiment of the present invention, the support base is formed with at least one conductor positioning element for mounting the coupling element, and the coupling element is formed with at least one mounting hole for being mounted to the at least one conductor positioning element, the cylindrical front portion of the support base has a positioning stage radially formed on an outer wall thereof for matching with the cigarette body, the support base is mounted into the cigarette body, and the outer wall of the cylindrical front portion is snugly mounted to the inner wall of the cigarette body and positioned by the positioning stage.

According to another embodiment of the present invention, the connector is a universal serial bus (USB) connector, a peg-type or a pin-type connector, the connector is substantially cylindrical and defined with the first accommodating chamber and a through hole therein, the outer wall of the support base is formed with a conductor slit, and the conductor passes through the through hole from the first accommodating chamber along the conductor slit to electrically connect the control circuit unit.

According to another embodiment of the present invention, the cigarette body has an inhaling rod and a battery rod, the inhaling rod comprises an inhaling cylinder, an inhaling nozzle, an atomizer disposed in the inhaling cylinder, and a cigarette liquid storing cup for storing a cigarette liquid, the battery rod comprises a barrel, a battery installed in the barrel, and a bottom case mounted to a bottom of the barrel, the connecting device is disposed in the barrel, and the bottom case covers the bottom of the barrel so as to seal the connecting device in the end of the barrel.

According to another embodiment of the present invention, the cigarette liquid storing cup comprises a cup seat and a cup lid both are spaced apart from each other at a predetermined space and are snugly mounted onto inner walls of the inhaling cylinder, a guiding tube and a liquid storing element both are disposed in between the cup seat and the cup lid, the guiding tube is being internally hollow with two opposite ends thereof open to outside, and the atomizer is fixedly held by the guiding tube, a positioning tube is mounted onto outer walls of the guiding tube for preventing the atomizer from moving in an axial direction of the guiding tube, the positioning tube and the guiding tube are in interference fit with each other, and the positioning tube abuts against the atomizer.

According to another embodiment of the present invention, the cup seat has a cylindrical cup structure, a positioning pillar formed in the cup seat for positioning the guiding tube, and a round internal chamber for receiving the liquid storing element; the cup lid having a substantially cylindrical lid structure, a circular internal cavity formed in the cup lid for receiving the guiding tube, a plurality of air vents formed on a side wall of the he cup lid, a plurality of ribs formed on an upper wall of the cup lid in the circular internal cavity, each of the plurality of ribs having a width smaller than an inner diameter of the guiding tube, a bottom of the cup lid extending radially outward to form a flange portion, wherein the flange portion is serrated and has multiple serrations, the serrations having gaps formed therebetween for enabling flow of the cigarette liquid to the cigarette liquid storing cup, two opposite ends of the guiding tube are respectively positioned by the upper wall of the cup lid and the positioning pillar of the cup seat, and the flange portion is disposed with respect to the round internal chamber of the cup seat for positioning two opposite ends of the liquid storing element.

According to another embodiment of the present invention, the inhaling rod and the battery rod are respectively installed with a male connecting element and a female connector so as to enable the inhaling rod and the battery rod to be plugged with each other, and the male and female connecting elements respectively have atomizer electrode elements and battery electrode elements, whereby the atomizer electrode element and the battery electrode element are in flexibly contact with each other to realize electrical connection.

According to another embodiment of the present invention, the male connecting element is substantially shaped as a round cover being internally hollow and has multiple air inlets radially formed at a bottom of the male connecting element, and a positioning step is formed by extending outward radially from the bottom of the male connecting element for abutting against a bottom of the inhaling cylinder, an first accommodating chamber is formed on the bottom of the male connecting element for being snugy mounted onto the battery rod, electrode slots are formed on a middle portion of the male connecting element and penetrate the male connecting element in an axialdirection thereof, and a positioning projection is radially formed on the bottom of the male connecting element and extends outward in the axial direction.

According to another embodiment of the present invention, the female connecting element is substantially shaped as a round cover and hasa side wall, an upper wall, an internal chamber defined by te side wall and the upper wall, a locating step is formed on the side wall adjacent to the upper wall and extends outward radially from the female connecting elemnt for fitting to the battery rod, an inserting peg is disposed on the upper wall and extends outward in an axial direction of the female connecting element for being inserted into the first accommodating chamber of the male connecting element, the upper wall is concavel formed with a plurality of positioning grooves for positioning the positioning projectionof the male connecting element, threading holes are formed in and penetrate the female connecting eleent in the axial direction, two electrode slits extend in the axial direction for positioning the battery electrode elements, and two internal channels for the insertion of the atomizer electrode elements with which the battery electrode elements re in flexible contact in the internal channels.

According to another embodiment of the present invention, the atomizer comprises at least one heating element, the atomizer electrode elements includes an atomizer first electrode element and an atomizer second electrode element respectively electrically connect with positive and negative electrodes of the at least one heating element, the battery electrode elements includes a battery first electrode element and a battery second electrode element respectively electrically connect with positive and negative electrodes of the battery, and the atomizer first and second electrode elements and the battery first and second electrode elements are all made of flexible metal conductive sheets.

According to another embodiment of the present invention, the atomizer first electrode element is a metal conductive sheet being flexibly deformable and is formed with a soldering plate of a soldering hole at one end thereof, a flexible contact plate being flexibly deformable and formed at another end of the atomizer first electrode element, and an engaging plate being flexibly deformable and formed at a middle of the atomizer first electrode element for engaging with the male connecting element; and the atomizer second electrode element has a structure same as that of the atomizer first electrode element.

According to another embodiment of the present invention, the battery first electrode element is a metal conductive sheet being flexibly deformable and is formed with a soldering plate of a soldering hole at one end thereof, a flexible contact plate being flexibly deformable and formed at another end of the battery fist electrode element, and an engaging plate being flexibly deformable and formed at a middle of the battery fist electrode element for engaging with the female connecting element; and the battery second electrode element has a structure same as that of the battery first electrode element.

According to another embodiment of the present invention, the atomizer first and second electrode elements and the battery first and second electrode elements each is formed with a soldering plate having a soldering hole at one end thereof, a flexible contact plate being flexibly deformable and formed at another end thereof, and an engaging plate being flexibly deformable and formed at a middle for engaging with corresponding male or female connecting element, the atomizer first and second electrode elements respectively electrically connect the negative electrodes of the at least one heating element through the soldering plates and soldering holes, the battery first and second electrode elements respectively electrically connect the positive and negative electrodes of the battery through the soldering plates and soldering holes, and the atomizer first and second electrode elements are in flexible contact with the battery first and second electrode elements through the flexible contact plates thereof to realize electrical connection.

Accordingly, the present invention has the advantages as follows: first, because the USB connector and the conductor are disposed in the electronic cigarette, the electronic cigarette is convenient to use and is aesthetic.

Secondly, the support base defines a first accommodating chamber formed therein for accommodating the USB connector and the conductor, whereby the USB connector and the conductor are freely to move therein.

Thirdly, the inhaling rod and the battery rod are pluggable to each other which enables a convenient and quick assembly or disassembly.

Fourthly, the positive and negative electrodes of the atomizer electrically connect the positive and negative electrodes of the battery in a manner that the atomizer first and second electrode elements are in flexibly contact with the battery first and second electrode elements, whereby the technical craft is being simplified, assembly is easy to be done, and electrical connection is reliable.

Fifthly, structure of the cup lid of the cigarette liquid storing cup is configured to efficiently discharge smoke and to enable a quickly flow of a cigarette liquid to the cigarette liquid storing cup.

Finally, a positioning tube is mounted onto outer walls of the guiding tube so as to further position and hold the atomizer in the guiding tube

Detailed description of the present invention is given below in conjunction with appendix drawings.

### BRIEF DESCRIPTION OF THE DRAWINGS

FIG. 1 is a front elevational view of an electronic cigarette of the present invention;
FIG. 2 is a cross-sectional view taken along line A-A in FIG. 1;
FIG. 3 is an enlarged view of a M portion indicated in FIG. 2;
FIG. 4 is an exploded view of an inhaling rod of the electronic cigarette of the present invention;
FIG. 5 is an exploded view of a battery rod of the electronic cigarette of the present invention;
FIG. 6 is a first perspective view of a cup lid of a cigarette liquid storing cup of the inhaling rod of the present invention;
FIG. 7 is a second perspective view of the cup lid of the cigarette liquid storing cup of the inhaling rod of the present invention;
FIG. 8 is a cross-sectional view of a cup seat of the cigarette liquid storing cup of the inhaling rod of the present invention;
FIG. 9 is a perspective view of a male connecting element of the inhaling rod of the present invention;
FIG. 10 is a cross-sectional view of the male connecting element of the inhaling rod of the present invention;
FIG. 11 is a perspective view of a female connecting element of the inhaling rod of the present invention;
FIG. 12 is a cross-sectional view of the female connecting element of the inhaling rod of the present invention;
FIG. 13 is a perspective view of an atomizer first electrode element of the inhaling rod of the present invention;
FIG. 14 is a perspective view of a battery first electrode element of the battery rod of the present invention;
FIG. 15 is a first perspective view of a support base of the battery rod of the present invention;
FIG. 16 is a second perspective view of the support base of the battery rod of the present invention;
FIG. 17 is a schematic view showing a state that a Connecting device is mounted in the support base according to the present invention;
FIG. 18 is a cross-sectional view of FIG. 17;
FIG. 19 is a schematic view showing a state that the connecting device is pulled out of the support base according to the present invention;
FIG. 20 is a cross-sectional view of FIG. 19;
FIG. 21 is a schematic view showing a state that a bottom case is pulled out of the battery rod according to the present invention;
FIG. 22 is a schematic view showing a state that the connecting device is pulled out of the battery rod according to the present invention; and
FIG. 23 is a schematic view showing a state that the inhaling rod is pulled out of the battery rod.

### DESCRIPTION OF THE PREFERRED EMBODIMENTS

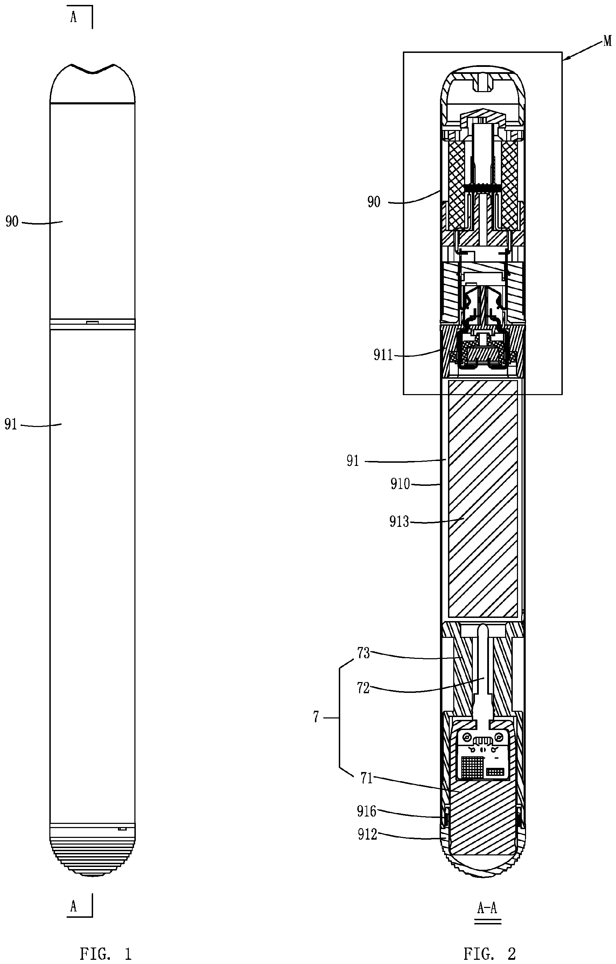

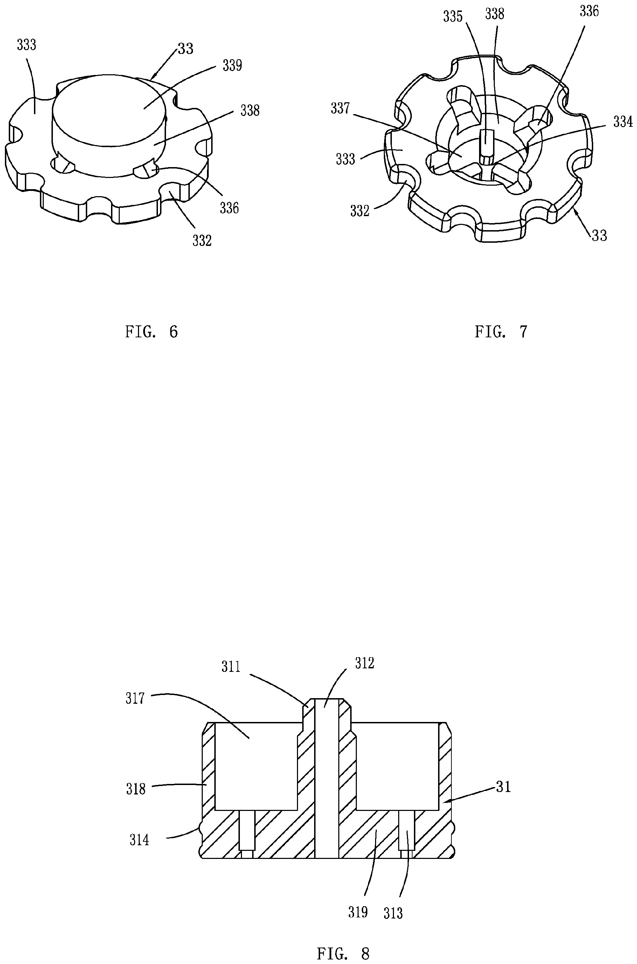

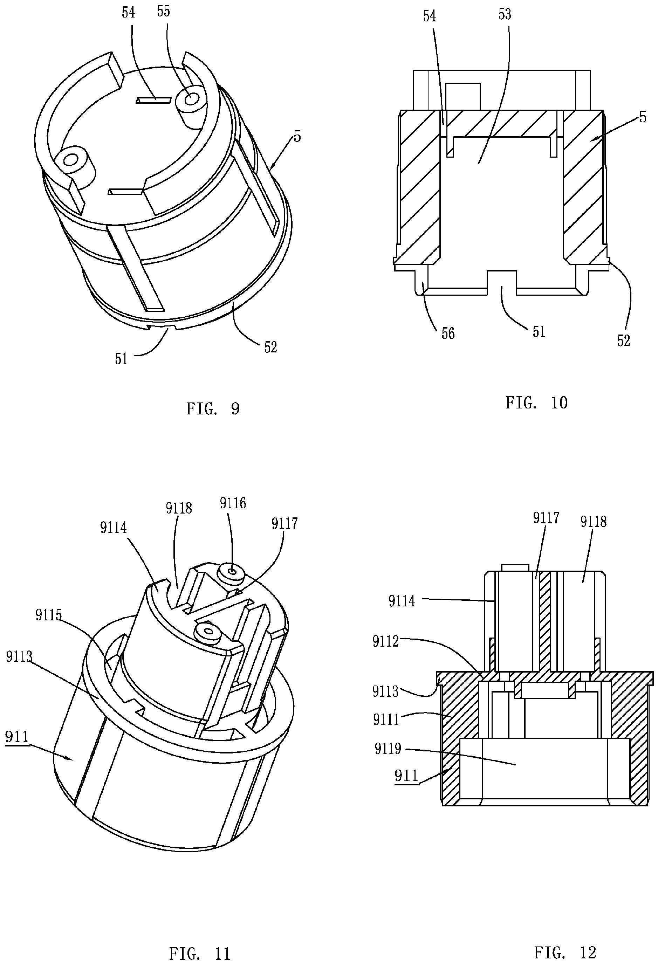

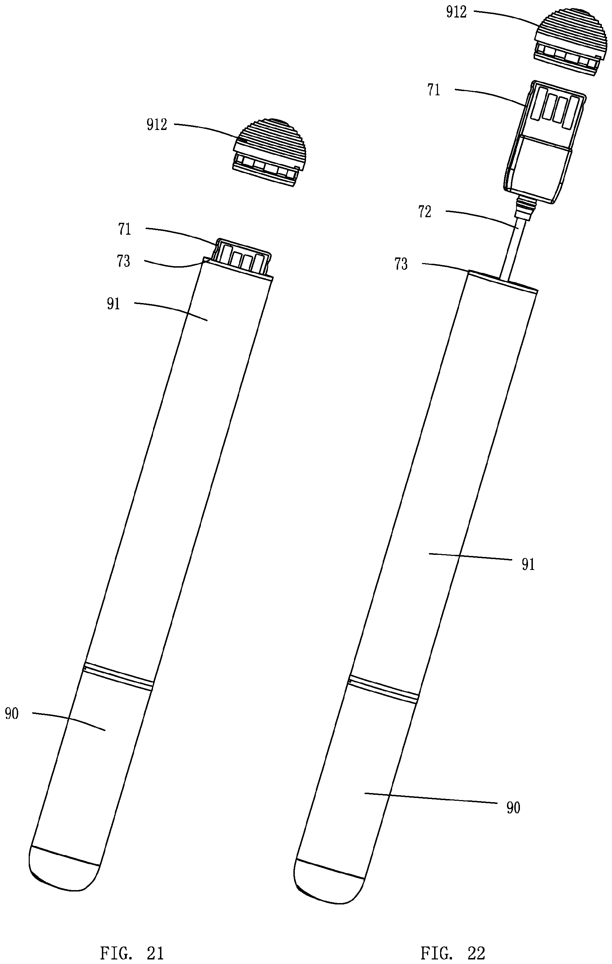

As shown in FIGS. 1 to 23, a first embodiment of the present invention is to provide an electronic cigarette, comprising a cigarette body having a inhaling rod 90 and a battery rod 91. The cigarette body is installed with a retractable connecting device 7. The connecting device 7 is retractably disposed in the cigarette body so as to be hidden therein. When not in use, the connecting device 7 is foldably retracted to be completely hidden within the cigarette body together. When in use, the connecting device 7 is being pulled away to extend to a predetermined length, whereby electrically connecting the electronic cigarette to an external electronic device or power. The connecting device 7 is intended to charge the electronic cigarette and to transmit data as well, such as multimedia data, so as to satisfy requirements of multi functions of the electronic cigarette. The connecting device 7 at least comprises a connector 71 and a conductor 72 of which a length is extendable. The connector 71 is one of a universal serial bus (USB) connector, a peg-type, a pin-type or other appropriate connector. A preferable embodiment described below is exemplified by the USB connector 71 in order to achieve the object of charging the electronic cigarette.

As shown in FIGS. 2 to 4, the inhaling rod 90 comprises an inhaling cylinder 1, an atomizing device 2 for atomizing a cigarette liquid, a cigarette liquid storing cup 3 for storing the cigarette liquid, an inhaling nozzle 4, and a male connecting element 5 for connecting the battery rod 91. In this embodiment, the inhaling cylinder 1 is a hollow elongated tube structure, and the inhaling nozzle 4 and the male connecting element 5 are mounted to opposite end portions of the inhaling cylinder 1.

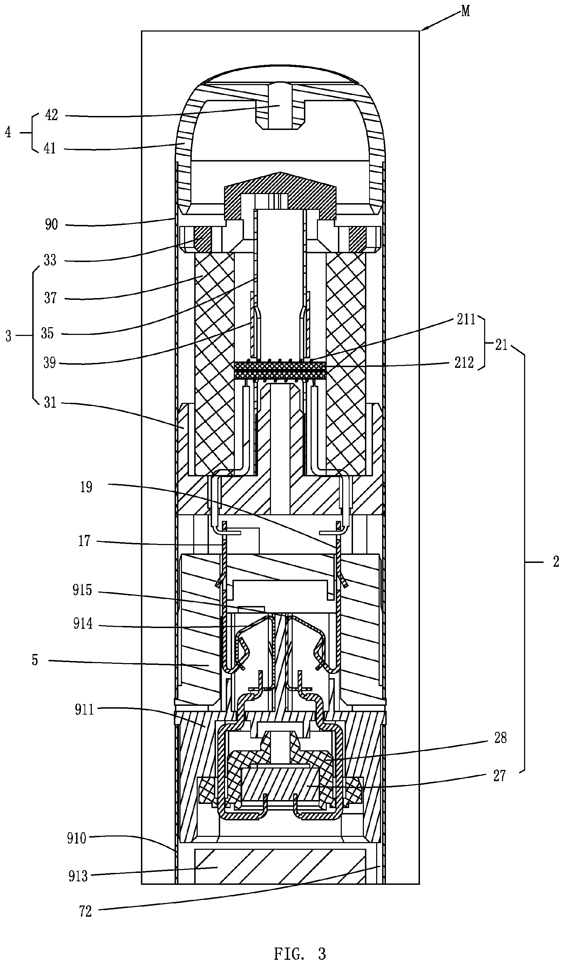

As shown in FIG. 3, the inhaling nozzle 4 has a substantially cylindrical body 41, which is internally hollow and has an inhaling hole 42 penetrating the cylindrical body 41 in an axial direction thereof. The inhaling nozzle 4 is made of a resilient material, and is detachably plugged into a top end of the inhaling cylinder 1.

As shown in FIGS. 3, 4, 9 and 10, the male connecting element 5, made of a flexible material, is disposed at a bottom of the inhaling cylinder 1 and has a shape fitted to the inhaling cylinder 1. cThe male connecting element 5 is substantially shaped as a round cover being internally hollow, and has multiple air inlets 51 formed at a bottom thereof along peripheral rims of the male connecting element 5 for allowing the outside air to flow in the inhaling cylinder 1. A positioning step 52 is formed by extending outward radially from the bottom of the male connecting element 5 for abutting against the bottom of the inhaling cylinder 1. A first accommodating chamber 53 is formed on the bottom of the male connecting element 5 for being snugly mounted onto the battery rod 91. Electrode slots 54 and air vents 55 are respectively formed on a middle portion of the male connecting element 5 and penetrate the male connecting element 5 in an axial direction thereof. A positioning projection 56 is formed on the bottom of the male connecting element 5 and extends outward in the axial direction for being inserted into the battery rod 91.

As shown in FIGS. 3, 4 and 13, the inhaling cylinder 1 further comprises an atomizer first electrode element 17 and an atomizer second electrode element 19 for electrically connecting electrodes of the battery rod 91. Both of the atomizer first and second electrode elements 17 and 19 are disposed in the male connecting element 5. The atomizer first electrode element 17 is a flexibly deformable metal sheet and has a soldering plate 171 at one end thereof, the soldering plate 171 defining a soldering hole, a flexible contact plate 172 being flexibly deformable and formed at another end thereof, and an engaging plate 173 being flexibly deformable and formed at a middle of the atomizer first electrode element 17 for engaging with the male connecting element 5. The atomizer second electrode element 19 has a structure same as that of the atomizer first electrode element 17.

As shown in FIGS. 3 to 5, the atomizing device 2 comprises an atomizer 21 installed in the inhaling rod 90, an atomizer switch27, a switch base 28, and a control circuit unit for controlling the atomizer 21.

The atomizer 21 is intended to atomize the cigarette liquid to be smoke, and comprises a heating element 211 and fiber elements 212. The heating element 211 is wound around the fiber elements 212 and held in a cigarette liquid storing cup 3. The fiber elements 212 are intended to absorb the cigarette liquid for the heating element 211 to heat and atomize. The fiber elements 212 function as a sponge that is capable of absorbing and preserving water, and are made of glass fiber or a material having characters of absorbing and isolating liquid, such as cotton, and have a tubular shape. In this embodiment, the number of the fiber elements 212 is three (not limited thereby). The three fiber elements 212 as a whole are disposed in the cigarette liquid storing cup 3. The heating element 211 is wound around outer surfaces of the whole three fiber element 212, wherein two opposite ends of the heating element 211 respectively extend out of the cigarette liquid storing cup 3 for electrically connecting with the atomizer first electrode element 17 and the atomizer second electrode element 19 of the male connecting element 5.

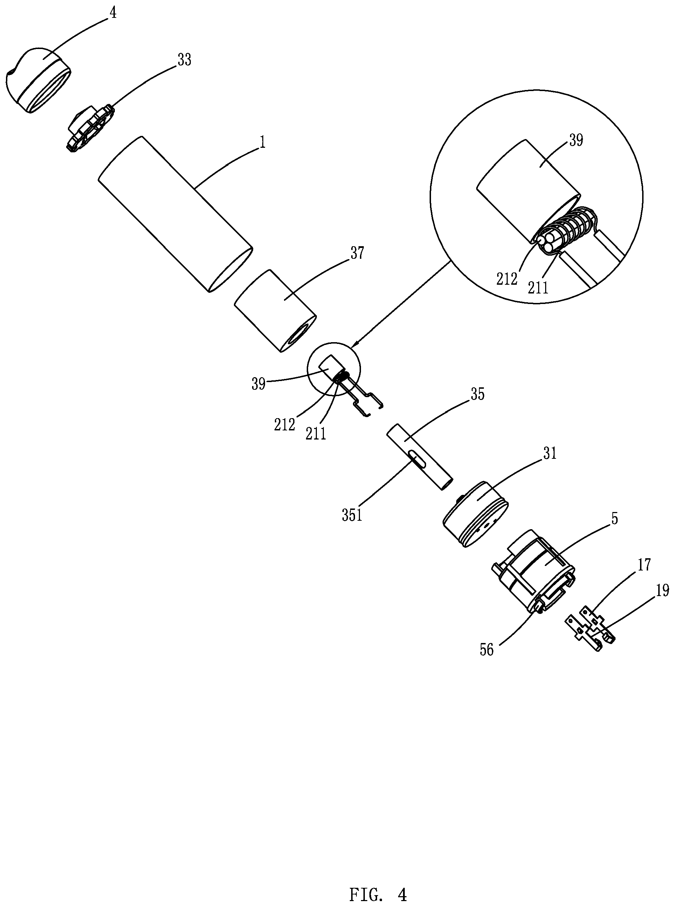

As shown in FIGS. 3 and 4, the cigarette liquid storing cup 3 comprises a cup seat 31, a cup lid 33, a guiding tube 35, a liquid storing element 37, and a positioning tube 39. The cup seat 31 and cup lid 33 both are spaced apart from each other at a predetermined space and are in snug-fit engagement with inner walls of the inhaling cylinder 1. The guiding tube 35 is mounted between the cup seat 31 and the cup lid 33. The liquid storing element 37 is disposed outside the guiding tube 35 between the cup seat 31 and the cup lid 33. The positioning tube 39 is intended to position the atomizer 21.

In this embodiment, the cup seat 31 (referring to FIGS. 3, 4 and 8) has a cylindrical cup structure, a round side wall 318, a round cup bottom 319, a positioning pillar 311 extends in an axial direction of cup seat 31 from a middle of the cup bottom 319, wherein the round side wall 318 and the positioning pillar 311 cooperatively define a round internal chamber 317. An air channel 312 is formed in the positioning pillar 311 and penetrates the positioning pillar 311 and the bottom cup 319 in its axial direction. A plurality of threading channels 313 are formed on and penetrate the cup bottom 319 for the heating element 211 to pass through. An outer surface of the side wall 318 is formed with a jam-fit ring 314. The cup seat 31 is snugly fitted to an inner wall of the inhaling cylinder 1 with the side wall 318 and the jam-fit ring 314.

The cup lid 33 (referring to FIGS. 3, 4, 6 and 7) is made of silica gel, and has a shape and size corresponding to the inner wall of the inhaling cylinder 1. In this embodiment, the cup lid 33 is a hollow cylindrical lid structure, and has a side wall 338 and an upper wall 339 both cooperatively define a circular internal cavity 337 having an inner diameter greater than an outer diameter of the guiding tube 35 in order for flow of smoke. A plurality of air vents 336 are formed on a side wall of the cup lid 33 and communicate with the circular internal cavity 337. A plurality of ribs 335 are formed on the upper wall 339 and located in the circular internal cavity 337. In this embodiment, there are three ribs 335 radially disposed on a middle portion of the upper wall 339, whereby when the guiding tube 35 is engaged with the ribs 224, a top end of the guiding tube 35 and the upper wall 339 jointly form a gap as a venting path 334 communicating with the circular internal cavity 337. A bottom of the side wall 338 extends radially outward to form a flange portion 333 which has multiple arc cutouts 332 and thus render the flange portion 333 serrated. An outer diameter of the flange portion 333 is slightly greater than an inner diameter of the inhaling cylinder 1. The cup lid 33 is snugly fitted with the inner wall of the inhaling cylinder 1 through the flange portion 333. The circular internal cavity 337 of the cup lid 33 corresponds to the positioning pillar 311 of the cup seat 31 and both respectively position the opposite ends of the guiding tube 35. The flange portion 333 of the cup lid 33 and the round internal chamber 317 of the cup seat 31 respectively fix two opposite ends of the liquid storing element 37. When the cigarette liquid is running out, pull out the inhaling nozzle 4 and remain the cup lid 33, and the cigarette liquid can be refilled in the cigarette liquid storing cup 3 from the arc cutouts 332 of the cup lid 33. The cigarette liquid is absorbed by the liquid storing element 37 and is capable of being repeatedly refilled in a convenient way.

The guiding tube 35 (referring to FIGS. 3 and 4) is intended to support the liquid storing element 37 and the fiber elements 212 and limit the height of the cigarette liquid storing cup 3, and further functions as a path to outside of the inhaling cylinder 1 for the flow of smoke produced by the atomizer 21 atomizing the cigarette liquid. In this embodiment, the guiding tube 35 is an insulating hollow cylinder, made of a plastic or fiber material, such as a fiberglass sleeve. The guiding tube 35 has top and bottom ends, wherein the top end is mounted in the circular internal cavity 337 of the cup lid 33, and the bottom end is snugly mounted to the positioning pillar 311 of the cup seat 31. The guiding tube 35 is formed with a retaining slot 351 penetrating side walls of the guiding tube 35 in a radial direction for retaining the fiber elements 212. The fiber elements 212 are fixed in the retaining slot 351 with two ends of the fiber elements 212 abutting against the liquid storing element 37 so as to absorb the cigarette liquid for the heating element 211 to atomize.

The liquid storing element 37 (referring to FIGS. 3 and 4) is intended to absorb and store the cigarette liquid in the cigarette liquid storing cup 3 so as to allow the atomizer 21 to atomize the cigarette liquid. The liquid storing element 37 functions as a sponge that is capable of absorbing and preserving water, and is made of glass fiber or a material having characters of absorbing and isolating liquid, such as cotton. The liquid storing element 37 is a hollow cylindrical structure and is disposed outside the guiding tube 35. One end of the liquid storing element 37 is inserted in the round internal chamber 317 of the cup seat 31 and the other end abuts against the bottom of the cup lid 33 such as the liquid storing element 37 is disposed between the cup seat 31 and the cup lid 33. Two ends of the fiber elements 212 abut against inner walls of the liquid storing element 37 to absorb the cigarette liquid for the heating element 211 to atomize.

The positioning tube 39 (referring to FIGS. 3 and 4) is intended to locate a position of the atomizer 21 with respect to the guiding tube 354. The positioning tube 39 is an insulating hollow cylinder being mountable onto outer walls of the guiding tube 35, and is made of a plastic or fiber material, such as a fiberglass sleeve. The positioning tube 39 and the guiding tube 35 are in interference fit with each other. A bottom of the positioning tube 39 abuts against the atomizer 21 to prevent the atomizer 21 from moving in an axial direction of the guiding tube 35.

In this embodiment, the cigarette liquid storing cup 3 defines a smoke path therein that is formed by the air channel 312 of the cup seat 31, a hollow passageway of the guiding tube 35, the venting path 334 of the cup lid 33, the circular internal cavity 337 and air vents 336 all together.

As shown in FIGS. 2, 3 and 5, the battery rod 91 comprises a barrel 910, a female connecting element 911 and a bottom case 912 respectively mounted to opposite ends of the barrel 910, a battery 913 installed in the barrel 910, and a battery first electrode element 914 and a battery second electrode 915 electrically connected to two electrodes of the battery 913.

As shown in FIGS. 3, 11 and 12, the female connecting element 911 is configured to mate with the male connecting element 5, and is made of a flexible plastic material. The female connecting element 911 is mounted to one end of the male connecting element 5 so as to connect the inhaling rod 90 and the battery rod 91. The female connecting element 911 is substantially shaped as a round cover, and comprises a side wall 9111, an upper wall 9112, an internal chamber 9119 is defined by the side wall 9111 and the upper wall 9112, a locating step 9113 is formed on the side wall 9111 adjacent to the upper wall 9112 and extends outward radially from the female connecting element 911 for fitting to the barrel 910, and an inserting peg 9114 is disposed on the upper wall 9112 and extends outward in an axial direction of the female connecting element 911 for being inserted into the first accommodating chamber 53 of the male connecting element 5. The upper wall 9112 is concavely formed with a plurality of positioning grooves 9115 for receiving the positioning projection 56 of the male connecting element 5. Threading holes 9116 are formed in and penetrate the female connecting element 911 in the axial direction, and two electrode slits 9117 and internal channels 9118 extend in the axial direction for positioning the battery first electrode element 914 and the battery second electrode element 915. The two internal channels 9118 for the insertion of the atomizer first and second electrode elements 17 and 19 with which the battery first and second electrode elements 914 and 915 are in flexible contact in the internal channels 9118.

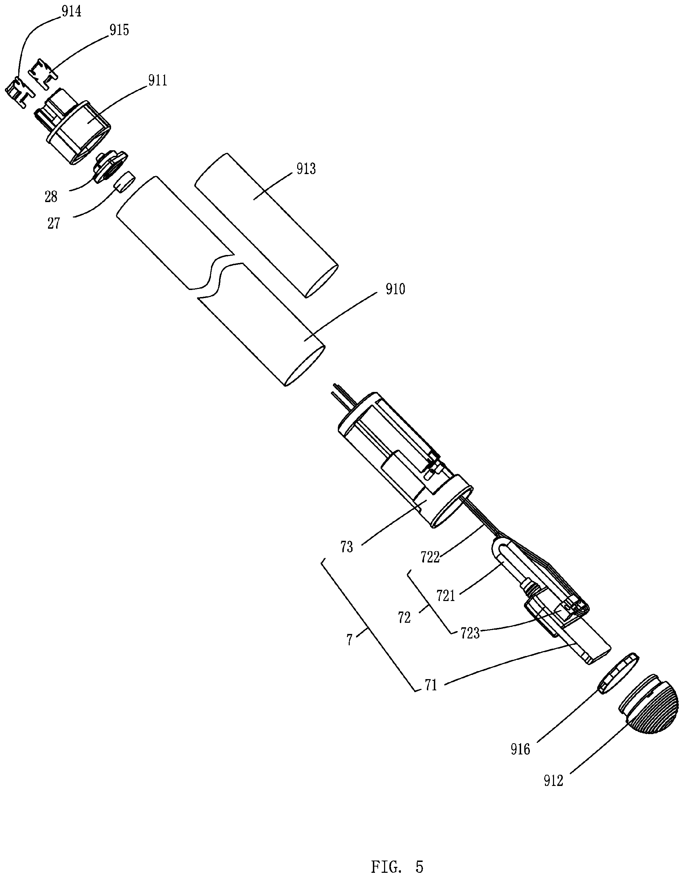

As shown in FIGS. 2 and 5, the bottom case 912 is mounted to the bottom end of the barrel 910 for sealing the USB connector 71. The bottom case 912 is further equipped with a sealing ring 916. It is understandable that to prevent the bottom case 912 from coming off the barrel 910 or lost after being pulled away, the bottom case 912 is further coupled with a coupling strap to connect the barrel 910. The coupling strap is capable of being disposed in the barrel 910 or integrally formed with a connecting portion or other connecting structure in order to mount the bottom case 912 onto the barrel.

As shown in FIGS. 3, 4 and 14, the battery first and second electrode elements 914 and 915 are respectively positioned in the electrode slits 9117. The battery first electrode element 914 is a flexibly deformable metal sheet, and has a soldering plate 9141 of a soldering hole at one end of the battery first electrode element 914, a flexible contact plate 9142 being flexibly deformable and formed at another end thereof, and an engaging plate 9143 being flexibly deformable and formed at a middle thereof for engaging with the female connecting element 911. The battery second electrode element 915 has a structure same as that of the battery first electrode element 914. When the inhaling rod 90 and the battery rod 91 are being plugged together, the battery first and second electrode elements 914 and 915 of the female connecting element 911 are in flexibly contact with the atomizer first and second electrode elements 17 and 19 whereby realizing electrical connection. The battery first and second electrode elements 914 and 915 respectively electrically connect the positive and negative electrodes of the battery 913 through the soldering plates 9141 and soldering holes. The atomizer first and second electrode elements 17 and 19 are in flexible contact with the battery first and second electrode elements 914 and 915 through the flexible contact plates thereof to realize electrical connection. Accordingly, the electronic cigarette is capable of being assembled conveniently with a simplified craft and providing a reliable electrical connection.

As shown in FIGS. 3 and 5, in this embodiment, the atomizer switch 27 and the switch base 28 are disposed in the battery rod 91. Specifically, the atomizer switch 27 is disposed in the switch base 28, while the switch base 28 is disposed in the internal chamber 9119 of the female connecting element 911. Two electrodes of the atomizer switch 27 are electrically connected to the battery first and second electrode elements 914 and 915 through two wires (not shown). The atomizer switch 27 is a pneumatic type switch that is switchable according to pneumatic vibration for power on or off. The atomizer switch 27 and the battery 913 are electrically connected to the control circuit unit.

As shown in FIGS. 2, 5, 15 to 22, the connecting device 7 has the connecting device 71, a conductor 72, and a support base 73. In this embodiment, the connecting device 7 is disposed in the battery rod 91.

The connecting device 71 is intended to connect an external power in order to charge the battery 911. The USB connector 71 is also capable of transmitting, downloading, or storing multimedia data when the electronic cigarette is equipped with portable storage devices, MP3, MP4 or other multimedia functions. The connecting device 71 is mounted in the battery rod 91 through the support base 73.

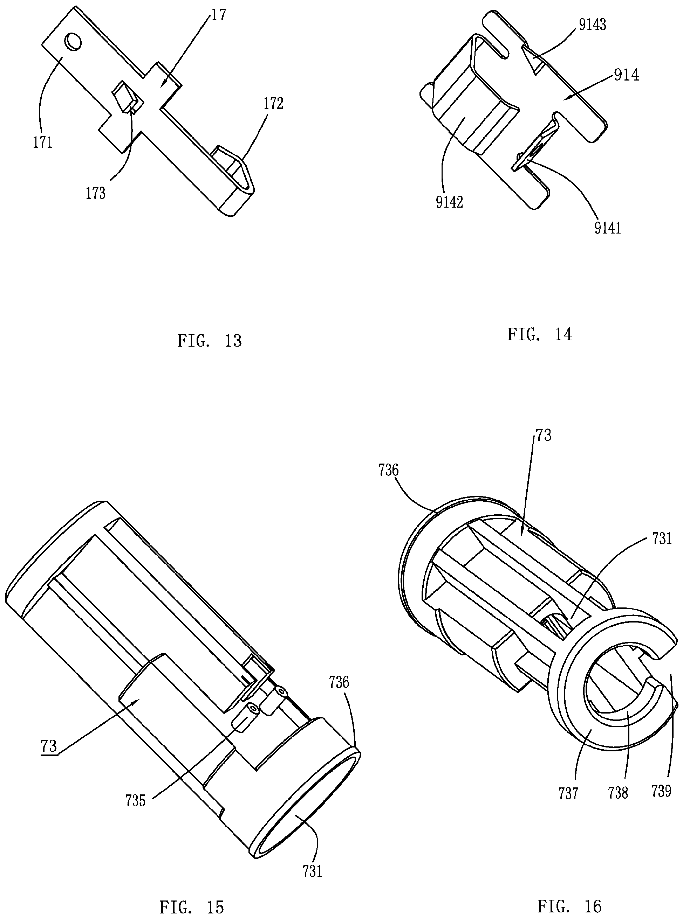

As shown in FIGS. 15 to 22, the support base 73 is being substantially cylindrical, and defines a first accommodating chamber 731 therein configured to receive the connecting device 71 and the conductor 72. The support base 73 is further formed with two conductor positioning elements 735 for positioning the conductor 72. The two conductor positioning elements 735 are disposed on a side wall of the support base 73 and each is exemplified by a screw post having internal screw threads. A positioning stage 736 is radially formed on an outer wall of a front portion of the support base 73 for matching with a bottom of the barrel 910. A bottom board 737 is formed at a bottom of the support base 73, and defines a through hole 738 and an opening 739 therein. Furthermore, the outer wall of the support base 73 is formed with a conductor slit (not labeled).

In this embodiment, the first accommodating chamber 731 comprises a front portion 7311, a middle portion 7312, and a rear portion 7313 all connected with each other in sequence. The front portion 7311 is cylindrical for allowing the USB connector 71 and the conductor 72 to extend out of or retract to the support base 73. The middle portion 7312 is being substantially cylindrical or other shapes and extends outwards from a bottom surface of the front portion 7311 in an axial direction of the support base 73. The USB connector 71 is wedged into the middle portion 7312. The rear portion 7313 is intended to accommodate the conductor 72 when the conductor 72 is being retracted. The rear portion 7313 is formed by the bottom board 737 and two parallel clamping boards (not shown) extending in the axial direction. The rear portion 7313 is hollow cylindrical or has other shapes for the purpose of accommodating the conductor 72 and allowing the conductor 72 to freely move therein. A penetrating hole is formed between the middle portion 7312 and the rear portion 7313. An outer wall of the front portion 7311 is fitted with the inner wall of the barrel 910. The support base 73 is mounted in the barrel 910 through a snug-fit of the outer wall of the front portion 7311 and the barrel 910, and is positioned by the positioning stage 736 (referring to FIGS. 2, 21, and 22). In this embodiment, the conductor positioning elements 735 are disposed on an outer side of the middle portion 7312 of the first accommodating chamber 731 and integrated with the support base 73.

One end of the conductor 72 is connected to the USB connector 71 so as to extend an applicable distance of the USB connector 71. Another end of the conductor 72 is connected to the control circuit unit in order for charging or other functions. The conductor 72 comprises a movable conductor 721, a fixing conductor 722, and a coupling element 723 for coupling the movable conductor 721 and the fixing conductor 722. The coupling element 723 is positioned by an inner wall or outer wall of the first accommodating chamber 731 so as to retain the fixing conductor 722. One end of the movable conductor 721 is electrically connected to the fixing conductor 722 through the coupling element 723. The other end of the movable conductor 721 is electrically connected to the USB connector 71. One end of the fixing conductor 722 is electrically connected to the movable conductor 721 through the coupling element 723. Another end of the fixing conductor 722 is attached on the outer wall of the support base 73 along the conductor slit to pass through the opening 739 and then electrically connect the control circuit unit. The fixing conductor 722 is fixed in the battery rod 91 without being retracted or extended. The coupling element 723 is substantially U-shaped and forms two mounting holes at opposite sides thereof for mounting the conductor positioning elements 735. The coupling element 723 is fixedly mounted onto the conductor positioning elements 735, with screws screwed in the conductor positioning elements 735. Alternatively, the coupling element 735 is capable of having other shapes and being mounted in other ways. Therefore, the fixing conductor 722 of the conductor 72 is being positioned and retained in place so as to prevent the electrical connection of the conductor 72 and the control circuit unit from being affected by the movable conductor 721 when the moveable conductor 721 is being drawn.

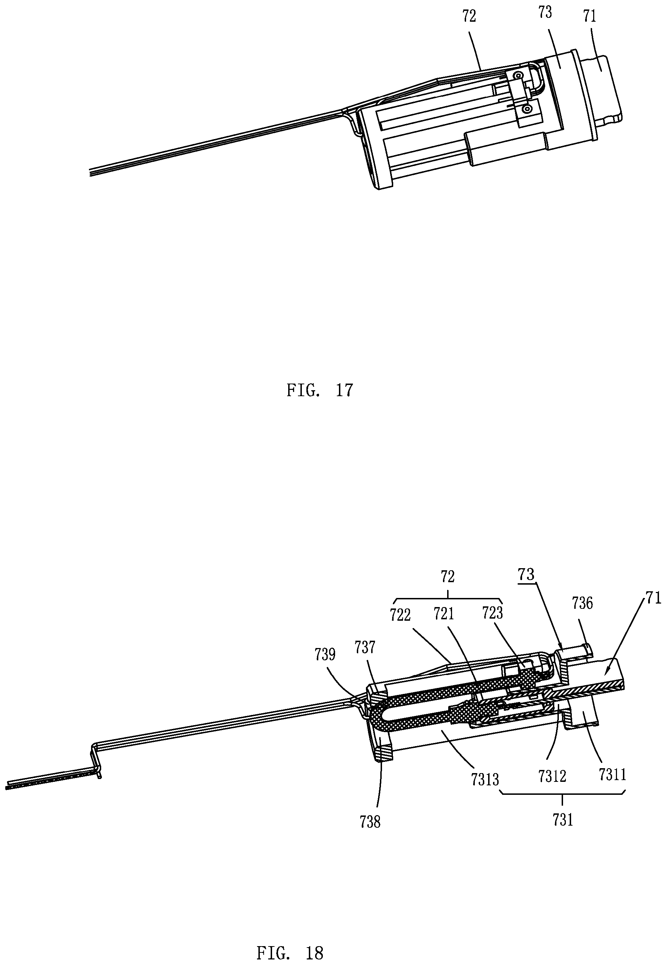

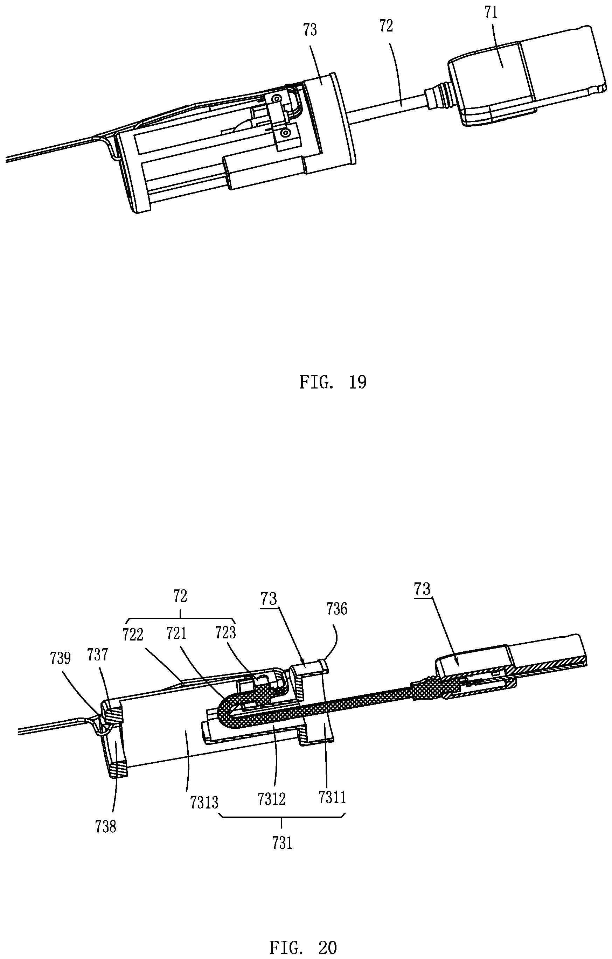

When the connecting device 7 is not in use, the USB connector 71 is manually depressed into the support base 73, and the bottom case 912 is being mounted, and thus the moveable conductor 721 is disposed in a conductor receiving slot 732 formed in the support base 73 (referring to FIG. 18). When the connecting device 7 is in use, first remove the bottom case 912, and then pull the USB connector 71 away of the support base 73 to come out of the barrel 910, whereby one end of the moveable conductor 721 reaches out of the barrel 910 from the first accommodating chamber 731 in conjunction with the USB connector 71 (referring to FIG. 20), and the movable conductor 721 brings the USB conductor 71 to connect with the external electronic device or power within a predetermined distance. In this embodiment, the number of the fixing conductor 722 is three, the three fixing conductors 722 respectively standing for a positive wire, a negative wire, and a signal wire for respectively connecting with respective electrodes of the control circuit unit. The connecting device 71 and the conductor 72 are disposed in the battery rod 91, and therefore enables a convenient usage and beautifies the electronic cigarette. The support base 73 defines the first accommodating chamber 731 for accommodating the USB connector 71 and the conductor 72 in a manner that the USB connector 71 and the conductor 72 are capable of freely moving therein.

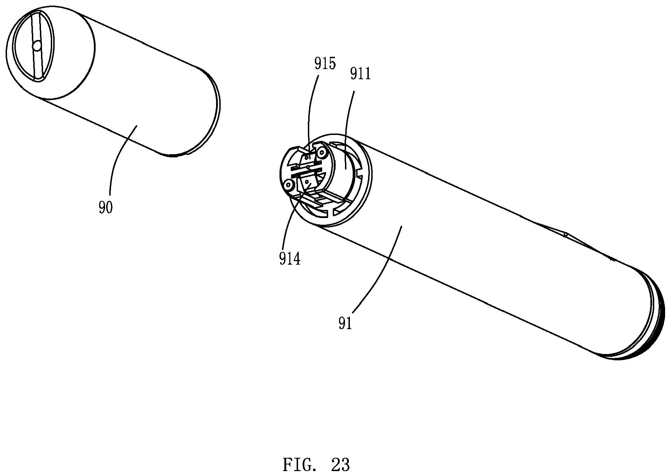

The inhaling rod 90 and the battery rod 91 are capable of being engaged or plugged together. As an embodiment shown in FIG. 23, the inhaling rod 90 and the battery rod 91 are detachably connected with each other through the male connecting element 5 snugly plugged with the female connecting element 911 in a convenient way. Alternatively, the electronic cigarette of the present invention is an one-piece element such that the inhaling cylinder 1 of the inhaling rod 90 and the barrel 910 of the battery rod 91 are integrally formed as an one-piece case body. Also, the inhaling cylinder 1, the barrel 910, and the inhaling nozzle 4 are all integrally formed.

It is understood that the invention may be embodied in other forms within the scope of the claims. Thus the present examples and embodiments are to be considered in all respects as illustrative, and not restrictive, of the invention defined by the claims.

## Claims

1. An electronic cigarette, comprising a cigarette body and a connecting device; the connecting device (7) comprising a connector (71) and a conductor (72) of which a length is extendable, one end of the conductor (72) connected to the connector (71), the other end of the conductor (72) electrically connected to a circuit control unit disposed in the cigarette body; whereby the connector electrically connects the electronic cigarette to an external electronic device or power;
**characterised in that**
the connecting device is retractably disposed in the cigarette body so as to be hidden therein; the conductor is capable of being retractably received in the cigarette body; the connector is capable of being retractably disposed in the cigarette body and extending out of the cigarette body through the retractable conductor to a pre-determined distance; wherein when the connecting device is not in use, the conductor is foldably retracted to be hidden within the cigarette body together with the connector, while the connecting device is in use, the connecting device is pulled away and the conductor extends to a predetermined length.

2. The electronic cigarette of claim 1, wherein the connecting device further comprises a support base disposed in the cigarette body for accommodating and positioning the connector, a first accommodating chamber is defined in the support base for accommodating the connector and the conductor, the first accommodating chamber having a wall body defining a cylindrical front portion for allowing the connector and the conductor to extend out of or retract to the support base, and the cylindrical front portion corresponds to an inner wall of the cigarette body.

3. The electronic cigarette of claim 2, wherein the conductor comprises a movable conductor and a fixing conductor both integrally connected with each other over a coupling element disposed in between the movable conductor and the fixing conductor, the coupling element is mounted to the support base, one end portion of the movable conductor is electrically connected to one end portion of the fixing conductor through the coupling element, the conductor is electrically connected to the connector through the one end portion of the movable conductor, and the conductor is electrically connected to a control circuit unit in the electronic cigarette through another end portion of the fixing conductor.

4. The electronic cigarette of claim 3, wherein the support base is formed with at least one conductor positioning element for mounting the coupling element, and the coupling element is formed with at least one mounting hole for being mounted to the at least one conductor positioning element, the cylindrical front portion of the support base has a positioning stage radially formed on an outer wall thereof for matching with the cigarette body, the support base is mounted into the cigarette body, and the outer wall of the cylindrical front portion is snugly mounted to the inner wall of the cigarette body and positioned by the positioning stage.

5. The electronic cigarette of claim 2, wherein the connector is a universal serial bus (USB) connector, a peg-type or a pin-type connector, the connector is substantially cylindrical and defined with the first accommodating chamber and a through hole therein, the outer wall of the support base is formed with a conductor slit, and the conductor passes through the through hole from the first accommodating chamber along the conductor slit to electrically connect the control circuit unit.

6. The electronic cigarette of claim 1, wherein the cigarette body has an inhaling rod and a battery rod, the inhaling rod comprises an inhaling cylinder, an inhaling nozzle, an atomizer disposed in the inhaling cylinder, and a cigarette liquid storing cup for storing a cigarette liquid, the battery rod comprises a barrel, a battery installed in the barrel, and a bottom case mounted to a bottom of the barrel, the connecting device is disposed in the barrel, and the bottom case covers the bottom of the barrel so as to seal the connecting device in the end of the barrel.

7. The electronic cigarette of claim 6, wherein the cigarette liquid storing cup comprises a cup seat and a cup lid both are spaced apart from each other at a predetermined space and are snugly mounted onto inner walls of the inhaling cylinder, a guiding tube and a liquid storing element both are disposed in between the cup seat and the cup lid, the guiding tube is being internally hollow with two opposite ends thereof open to outside, and the atomizer is fixedly held by the guiding tube, a positioning tube is mounted onto outer walls of the guiding tube for preventing the atomizer from moving in an axial direction of the guiding tube, the positioning tube and the guiding tube are in interference fit with each other, and the positioning tube abuts against the atomizer.

8. The electronic cigarette of claim 7, wherein the cup seat has a cylindrical cup structure, a positioning pillar formed in the cup seat for positioning the guiding tube, and a round internal chamber for receiving the liquid storing element; the cup lid having a substantially cylindrical lid structure, a circular internal cavity formed in the cup lid for receiving the guiding tube, a plurality of air vents formed on a side wall of the he cup lid, a plurality of ribs formed on an upper wall of the cup lid in the circular internal cavity, each of the plurality of ribs having a width smaller than an inner diameter of the guiding tube, a bottom of the cup lid extending radially outward to form a flange portion, wherein the flange portion is serrated and has multiple serrations, the serrations having gaps formed therebetween for enabling flow of the cigarette liquid to the cigarette liquid storing cup, two opposite ends of the guiding tube are respectively positioned by the upper wall of the cup lid and the positioning pillar of the cup seat, and the flange portion is disposed with respect to the round internal chamber of the cup seat for positioning two opposite ends of the liquid storing element.

9. The electronic cigarette of claim 6, wherein the inhaling rod and the battery rod are respectively installed with a male connecting element and a female connector so as to enable the inhaling rod and the battery rod to be plugged with each other, and the male and female connecting elements respectively have atomizer electrode elements and battery electrode elements, whereby the atomizer electrode element and the battery electrode element are in flexibly contact with each other to realize electrical connection.

10. The electronic cigarette of claim 9, wherein the male connecting element is substantially shaped as a round cover being internally hollow and has multiple air inlets radially formed at a bottom of the male connecting element, and a positioning step is formed by extending outward radially from the bottom of the male connecting element for abutting against a bottom of the inhaling cylinder, an first accommodating chamber is formed on the bottom of the male connecting element for being snugy mounted onto the battery rod, electrode slots are formed on a middle portion of the male connecting element and penetrate the male connecting element in an axial direction thereof, and a positioning projection is radially formed on the bottom of the male connecting element and extends outward in the axial direction.

11. The electronic cigarette of claim 10, wherein the female connecting element is substantially shaped as a round cover and has a side wall, an upper wall, an internal chamber defined by te side wall and the upper wall, a locating step is formed on the side wall adjacent to the upper wall and extends outward radially from the female connecting elemnt for fitting to the battery rod, an inserting peg is disposed on the upper wall and extends outward in an axial direction of the female connecting element for being inserted into the first accommodating chamber of the male connecting element, the upper wall is concavel formed with a plurality of positioning grooves for positioning the positioning projectionof the male connecting element, threading holes are formed in and penetrate the female connecting eleent in the axial direction, two electrode slits extend in the axial direction for positioning the battery electrode elements, and two internal channels for the insertion of the atomizer electrode elements with which the battery electrode elements re in flexible contact in the internal channels.

12. The electronic cigarette of claim 9, wherein the atomizer comprises at least one heating element, the atomizer electrode elements includes an atomizer first electrode element and an atomizer second electrode element respectively electrically connect with positive and negative electrodes of the at least one heating element, the battery electrode elements includes a battery first electrode element and a battery second electrode element respectively electrically connect with positive and negative electrodes of the battery, and the atomizer first and second electrode elements and the battery first and second electrode elements are all made of flexible metal conductive sheets.

13. The electronic cigarette of claim 12, the atomizer first electrode element is a metal conductive sheet being flexibly deformable and is formed with a soldering plate of a soldering hole at one end thereof, a flexible contact plate being flexibly deformable and formed at another end of the atomizer first electrode element, and an engaging plate being flexibly deformable and formed at a middle of the atomizer first electrode element for engaging with the male connecting element; and the atomizer second electrode element has a structure same as that of the atomizer first electrode element.

14. The electronic cigarette of claim 12, wherein the battery first electrode element is a metal conductive sheet being flexibly deformable and is formed with a soldering plate of a soldering hole at one end thereof, a flexible contact plate being flexibly deformable and formed at another end of the battery fist electrode element, and an engaging plate being flexibly deformable and formed at a middle of the battery fist electrode element for engaging with the female connecting element; and the battery second electrode element has a structure same as that of the battery first electrode element.

15. The electronic cigarette of claim 12, wherein the atomizer first and second electrode elements and the battery first and second electrode elements each is formed with a soldering plate having a soldering hole at one end thereof, a flexible contact plate being flexibly deformable and formed at another end thereof, and an engaging plate being flexibly deformable and formed at a middle for engaging with corresponding male or female connecting element, the atomizer first and second electrode elements respectively electrically connect the negative electrodes of the at least one heating element through the soldering plates and soldering holes, the battery first and second electrode elements respectively electrically connect the positive and negative electrodes of the battery through the soldering plates and soldering holes, and the atomizer first and second electrode elements are in flexible contact with the battery first and second electrode elements through the flexible contact plates thereof to realize electrical connection.

## Patentansprüche

1. Eine elektronische Zigarette, welche einen Zigarettenkörper und eine Verbindungsvorrichtung umfasst; wobei die Verbindungsvorrichtung (7) einen Stecker (71) und einen Leiter (72) umfasst, der in der Länge ausziehbar ist, wobei ein Ende des Leiters (72) mit dem Stecker (71) verbunden und das andere Ende des Leiters (72) elektrisch mit einer Schaltungs-Steuerungseinheit verbunden ist, welche in dem Zigarettenkörper angeordnet ist; wobei der Stecker die elektronische Zigarette elektrisch mit einem externen elektronischen Gerät oder einer Stromquelle verbindet; **dadurch gekennzeichnet, dass** die Verbindungsvorrichtung einschiebbar in dem Zigarettenkörper angeordnet ist, sodass sie darin verborgen werden kann; der Leiter einschiebbar in dem Zigarettenkörper aufgenommen werden kann; der Stecker einschiebbar in dem Zigarettenkörper angeordnet werden kann und sich durch den einschiebbaren Leiter aus dem Zigarettenkörper bis auf eine vorbestimmte Distanz heraus bewegen kann; worin, während die Verbindungsvorrichtung nicht benutzt wird, der Leiter faltbar zurückgezogen ist, um zusammen mit dem Stecker in dem Zigarettenkörper verborgen zu sein, und während die Verbindungsvorrichtung benutzt wird, die Verbindungsvorrichtung herausgezogen und der Leiter auf eine vorbestimmte Länge ausgezogen ist.

2. Die elektronische Zigarette nach Anspruch 1, wobei die Verbindungsvorrichtung weiter einen Aufnahmesockel umfasst, welcher in dem Zigarettenkörper angeordnet ist, um den Stecker aufzunehmen und in Position zu halten, eine erste Aufnahmekammer in dem Aufnahmesockel definiert ist, um den Stecker und den Leiter aufzunehmen, wobei die erste Aufnahmekammer einen Wandkörper aufweist, der einen zylinderförmigen Vorderabschnitt definiert, der es ermöglicht, dass sich der Stecker und der Leiter aus der zurückgezogenen Position hin zu dem Aufnahmesockel bewegen und wobei der zylinderförmigen Vorderabschnitt einer inneren Wand des Zigarettenkörpers entspricht.

3. Die elektronische Zigarette nach Anspruch 2, wobei der Leiter einen beweglichen Leiter und einen Fixierungsleiter umfasst, welche über ein Kupplungselement beide fest miteinander verbunden sind, das zwischen dem beweglichen Leiter und dem Fixierungsleiter angeordnet ist, wobei das Kupplungselement auf dem Aufnahmesockel befestigt ist, ein Ende des beweglichen Leiters durch das Kupplungselement elektrisch mit einem Endabschnitt des Fixierungsleiters verbunden ist, der Leiter durch den einen Endabschnitt des beweglichen Leiters elektrisch mit dem Stecker verbunden ist und der Leiter durch einen anderen Endabschnitt des Fixierungsleiters elektrisch mit einer Schaltungs-Steuerungseinheit in der elektronischen Zigarette verbunden ist.

4. Die elektronische Zigarette nach Anspruch 3, wobei der Aufnahmesockel mit mindestens einem Leiterpositionierungselement für die Anbringung des Kupplungselements ausgeformt ist, und das Kupplungselement mit mindestens einer Befestigungsbohrung für die Befestigung auf dem mindestens einem Leiterpositionierungselement ausgeformt ist, wobei der zylinderförmige Vorderabschnitt des Aufnahmesockels eine Positionierungsebene aufweist, welche an einer Außenwand desselben radial und passend zu dem Zigarettenkörper ausgebildet ist, der Aufnahmesockel in dem Zigarettenkörper angebracht wird und die Außenwand des zylinderförmigen Vorderabschnitts eng an der Innenwand des Zigarettenkörpers angebracht und von der Positionierungsebene in Position gehalten wird.

5. Die elektronische Zigarette nach Anspruch 2, wobei der Stecker ein Universal Serial Bus (USB)-Stecker, ein zapfenartiger oder polartiger Stecker ist, der Stecker im Wesentlichen zylindrisch und mit der ersten Aufnahmekammer und einer durchgehenden Öffnung darin definiert ist, die Außenwand des Aufnahmesockels mit einem Leiterschlitz ausgeformt ist und der Leiter durch das Durchgangsloch von der ersten Aufnahmekammer entlang des Leiterschlitzes tritt, um eine elektrische Verbindung zur Schaltungs-Steuerungseinheit herzustellen.

6. Die elektronische Zigarette nach Anspruch 1, wobei der Zigarettenkörper einen Inhalationsstab und einen Batteriestab aufweist, wobei der Inhalationsstab einen Inhalationszylinder, eine Inhalationsdüse, einen Zerstäuber, der in dem Inhalationszylinder angeordnet ist, und einen Zigarettenflüssigkeitsbehälter für die Aufbewahrung einer Zigarettenflüssigkeit umfasst, wobei der Batteriestab einen Zylinder, eine in dem Zylinder angeordnete Batterie und eine Unterschale umfasst, die an der Unterseite des Zylinders angebracht ist, die Verbindungsvorrichtung in dem Zylinder angeordnet ist, und die Unterschale den Boden des Zylinders derart abdeckt, dass die Verbindungsvorrichtung im Endstück des Zylinders eingeschlossen ist.

7. Die elektronische Zigarette nach Anspruch 6, wobei der Zigarettenflüssigkeitsbehälter eine Behälterhalterung und eine Behälterabdeckung umfasst, welche beide in einem vorbestimmten Abstand voneinander angeordnet und eng an den Innenwänden des Inhalationszylinders angebracht sind, ein Führungsrohr und ein Flüssigkeitsspeicherelement, welche beide zwischen der Behälterhalterung und der Behälterabdeckung angeordnet sind, wobei das Führungsrohr innen hohl und an beiden Enden nach außen hin offen ist, und der Zerstäuber fest von dem Führungsrohr gehalten wird, ein Positionierungsrohr auf den Außenwänden des Führungsrohrs angebracht ist, um zu verhindern, dass der Zerstäuber sich in einer axialen Richtung des Führungsrohrs bewegt und das Positionierungsrohr und das Führungsrohr passgenau ineinander greifen und das Positionierungsrohr an dem Zerstäuber endet.

8. Die elektronische Zigarette nach Anspruch 7, wobei die Behälterhalterung eine zylindrische Behälterstruktur aufweist, eine Positionierungsstütze, die in dem Behälterhalterung ausgebildet ist, um das Führungsrohr in Position zu halten, und eine runde Innenkammer für die Aufnahme des Flüssigkeitsspeicherelements; wobei die Behälterabdeckung eine im Wesentlichen zylindrische Abdeckungsstruktur aufweist, einen runden internen Hohlraum, der in der Behälterabdeckung ausgeformt ist für die Aufnahme der Führungsrohrs, eine Vielzahl von Lüftungsöffnungen, welche an einer Seitenwand der Behälterabdeckung ausgebildet sind, eine Vielzahl von Rippen, die auf einer oberen Wand der Behälterabdeckung in dem runden internen Hohlraum ausgebildet sind, wobei jede aus der Vielzahl von Rippen eine Breite ausweist, die kleiner ist als ein Innendurchmesser des Führungsrohrs, wobei sich ein Boden der Behälterabdeckung radial nach außen erstreckt und einen Flanschabschnitt bildet, wobei der Flanschabschnitt gezahnt ist und mehrere Zähne aufweist, wobei zwischen den Zähnen Lücken ausgebildet sind, durch welche die Zigarettenflüssigkeit zu dem Zigarettenflüssigkeitsbehälter hinfließen kann, wobei zwei gegenüberliegende Enden des Führungsrohrs jeweils von der oberen Wand der Behälterabdeckung und der Positionierungsstütze der Behälterhalterung in Position gehalten werden, und der Flanschabschnitt mit Bezug zu der runden Innenkammer der Behälterhalterung angeordnet ist, um die beiden gegenüberliegenden Enden des Flüssigkeitsspeicherelements in Position zu halten.

9. Die elektronische Zigarette nach Anspruch 6, wobei der Inhalationsstab und der Batteriestab jeweils mit einem Stift-Verbindungselement und einem Buchsenstecker angebracht werden, sodass der Inhalationsstab und der Batteriestab ineinander gesteckt werden können, und wobei das Stift-Verbindungselement und der Buchsenstecker jeweils Zerstäuber-Elektrodenelemente und Batterie-Elektrodenelemente aufweisen, wobei das Zerstäuber-Elektrodenelement und das Batterie-Elektrodenelement in flexiblem Kontakt miteinander stehen, um eine elektrische Verbindung herzustellen.

10. Die elektronische Zigarette nach Anspruch 9, wobei das Stift-Verbindungselement im Wesentlichen als runde, innen hohle Abdeckung ausgeformt ist und mehrere Lufteinlässe aufweist, welche radial an einer Unterseite des Stift-Verbindungselements ausgeformt sind, und ein Positionierungsabsatz gebildet wird, indem er sich radial von der Unterseite des Stift-Verbindungselements nach außen erstreckt und so bis zur Unterseite des Inhalationszylinders reicht, eine erste Aufnahmekammer an der Unterseite des Stift-Verbindungselements ausgeformt ist, um ein eng anliegendes Anbringen an dem Batteriestab zu ermöglichen, in einem mittleren Abschnitt des Stift-Verbindungselements Elektrodenschlitze ausgebildet sind, die das Stift-Verbindungselement in axialer Richtung dessen durchdringen, und ein Positionierungsfortsatz radial auf der Unterseite des Stift-Verbindungselements ausgeformt ist und sich in axialer Richtung nach außen erstreckt.

11. Die elektronische Zigarette nach Anspruch 10, wobei das Buchsen-Verbindungselement im Wesentlichen als runde Abdeckung ausgeformt ist und eine Seitenwand, eine obere Wand, und eine Innenkammer aufweist, die von der Seitenwand und der oberen Wand definiert wird, und wobei an der an die obere Wand angrenzenden Seitenwand eine Lokalisierungsstufe ausgeformt ist, die sich radial von dem Buchsen-Verbindungselement nach außen erstreckt für die Einpassung des Batteriestabs, wobei ein Einführungszapfen an der oberen Wand angeordnet ist und sich in axialer Richtung des Buchsen-Verbindungselements nach außen erstreckt, damit er in die erste Aufnahmekammer des Stift-Verbindungselements eingeführt werden kann, wobei die obere Wand konkav ausgeformt und mit einer Vielzahl von Positionierungsrillen für das In-Position-Halten des Positionierungsfortsatzes des Stift-Verbindungselements ausgestattet ist, wobei in dem Buchsen-Verbindungselement Einführlöcher ausgeformt sind, die dieses in axialer Richtung durchdringen, wobei sich zwei Elektrodenschlitze für die Positionierung der Batterie-Elektrodenelemente in axialer Richtung erstrecken und zwei Innenkanäle für das Einführen der Zerstäuber-Elektrodenelemente, mit denen die Batterie-Elektrodenelemente in den Innenkanälen in flexiblem Kontakt stehen.

12. Die elektronische Zigarette nach Anspruch 9, wobei der Zerstäuber mindestens ein Heizelement umfasst, das Zerstäuber-Elektrodenelement ein erstes Zerstäuber-Elektrodenelement und ein zweites Zerstäuber-Elektrodenelement beinhaltet, welche jeweils elektrisch mit positiven und negativen Elektroden des mindestens einen Heizelements verbunden sind, wobei das Batterie-Elektrodenelement ein erstes Batterie-Elektrodenelement und ein zweites Batterie-Elektrodenelement beinhaltet, welche jeweils elektrisch mit positiven und negativen Elektroden der Batterie verbunden sind, und wobei das erste und das zweite Zerstäuber-Elektrodenelement sowie das erste und das zweite Batterie-Elektrodenelement allesamt aus flexiblen, leitfähigen Metallplatten hergestellt sind.

13. Die elektronische Zigarette nach Anspruch 12, wobei das erste Zerstäuber-Elektrodenelement eine leitfähige Metallplatte darstellt, die flexibel verformbar und mit einer Lötplatte eines Lötlochs an einem Ende dessen ausgebildet ist, eine flexible Kontaktplatte, die flexibel verformbar und an einem anderen Ende des ersten Zerstäuber-Elektrodenelements ausgebildet ist, und eine Verbindungsplatte, die flexibel verformbar und in einer Mitte des ersten Zerstäuber-Elektrodenelements ausgebildet ist für die Verbindung mit dem Stift-Verbindungselement; und wobei das zweite Zerstäuber-Elektrodenelement eine Struktur aufweist, die der des ersten Zerstäuber-Elektrodenelements entspricht.

14. Die elektronische Zigarette nach Anspruch 12, wobei das erste Batterie-Elektrodenelement eine leitfähige Metallplatte darstellt, die flexibel verformbar und mit einer Lötplatte eines Lötlochs an einem Ende dessen ausgebildet ist, eine flexible Kontaktplatte, die flexibel verformbar und an einem anderen Ende des ersten Batterie-Elektrodenelements ausgebildet ist, und eine Verbindungsplatte, die flexibel verformbar und in einer Mitte des ersten Batterie-Elektrodenelements ausgebildet ist für die Verbindung mit dem Stift-Verbindungselement; und wobei das zweite Batterie-Elektrodenelement eine Struktur aufweist, die der des ersten Batterie-Elektrodenelements entspricht.

15. Die elektronische Zigarette nach Anspruch 12, worin das erste und das zweite Zerstäuber-Elektrodenelement und das erste und das zweite Batterie-Elektrodenelement jeweils mit einer Lötplatte ausgebildet sind, an deren Ende sich ein Lötloch befindet, mit einer flexiblen Kontaktplatte, die flexibel verformbar und an einem anderen Ende davon ausgebildet ist, und einer Verbindungsplatte, die flexibel verformbar und in einer Mitte ausgebildet ist für die Verbindung mit dem entsprechenden Stift- oder Buchsen-Verbindungselement, wobei das erste und das zweite Zerstäuber-Elektrodenelement jeweils die negativen Elektroden des mindestens einen Heizelements durch die Lötplatten und Lötlöcher elektrisch verbinden, das erste und das zweite Batterie-Elektrodenelement jeweils die positiven und negativen Elektroden der Batterie durch die Lötplatten und Lötlöcher verbinden, und das erste und das zweite Zerstäuber-Elektrodenelement durch deren flexible Kontaktplatten in flexiblem Kontakt mit dem ersten und dem zweiten Batterie-Elektrodenelement sind, um eine elektrische Verbindung herzustellen.

## Revendications

1. Cigarette électronique, comprenant un corps de cigarette et un dispositif de connexion ;
le dispositif (7) de connexion comprenant un connecteur (71) et un conducteur (72) dont une longueur est extensible, une extrémité du conducteur (72) connectée au connecteur (71), l'autre extrémité du conducteur (72) électriquement connectée à une unité de commande de circuit disposée dans le corps de cigarette ; dans laquelle le connecteur connecte électriquement la cigarette électronique à un dispositif électronique ou une alimentation externe ;
**caractérisée en ce que**
le dispositif de connexion est disposé rétractable dans le corps de cigarette de manière à être caché dans celui-ci ; le conducteur est apte à être reçu rétractable dans le corps de cigarette ; le connecteur est apte à être disposé rétractable dans le corps de cigarette et à s'étendre hors du corps de cigarette par l'intermédiaire du conducteur rétractable d'une distance prédéterminée ; dans laquelle, lorsque le dispositif de connexion n'est pas en utilisation, le conducteur est rétracté plié pour être caché à l'intérieur du corps de cigarette ensemble avec le connecteur, lorsque le dispositif de connexion est en utilisation, le dispositif de connexion est sorti et le conducteur s'étend d'une longueur prédéterminée.

2. Cigarette électronique selon la revendication 1, dans laquelle le dispositif de connexion comprend en outre une base de support disposée dans le corps de cigarette pour recevoir et positionner le connecteur, une première chambre de réception est définie dans la base de support pour recevoir le connecteur et le conducteur, la première chambre de réception ayant un corps de paroi définissant une partie avant cylindrique pour permettre au connecteur et au conducteur de s'étendre hors de ou de se rétracter dans la base de support, et la partie avant cylindrique correspond à une paroi intérieure du corps de cigarette.

3. Cigarette électronique selon la revendication 2, dans laquelle le conducteur comprend un conducteur mobile et un conducteur de fixation tous les deux intégralement connectés l'un avec l'autre sur un élément de couplage disposé entre le conducteur mobile et le conducteur de fixation, l'élément de couplage est monté sur la base de support, une partie d'extrémité du conducteur mobile est électriquement connectée à une partie d'extrémité du conducteur de fixation par l'intermédiaire de l'élément de couplage, le conducteur est électriquement connecté au connecteur par l'intermédiaire de la partie d'extrémité du conducteur mobile, et le conducteur est électriquement connecté à une unité de circuit de commande dans la cigarette électronique par l'intermédiaire d'une autre partie d'extrémité du conducteur de fixation.

4. Cigarette électronique selon la revendication 3, dans laquelle la base de support est formée avec au moins un élément de positionnement de conducteur pour monter l'élément de couplage, et l'élément de couplage est formé avec au moins un trou de montage pour être monté sur l'au moins un élément de positionnement de conducteur, la partie avant cylindrique de la base de support a une platine de positionnement formée radialement sur une paroi extérieure de celle-ci pour correspondance avec le corps de cigarette, la base de support est montée dans le corps de cigarette, et la paroi extérieure de la partie avant cylindrique est montée de façon serrée sur la paroi intérieure du corps de cigarette et positionnée par la platine de positionnement.

5. Cigarette électronique selon la revendication 2, dans laquelle le connecteur est un connecteur bus série universel (USB), un connecteur de type à cheville ou de type à broche, le connecteur est sensiblement cylindrique et défini avec la première chambre de réception et un trou traversant dans celle-ci, la paroi extérieure de la base de support est formée avec une fente de conducteur, et le conducteur passe à travers le trou traversant depuis la première chambre de réception le long de la fente de conducteur pour se connecter électriquement à l'unité de circuit de commande.

6. Cigarette électronique selon la revendication 1, dans laquelle le corps de cigarette a une tige d'inhalation et une tige de batterie, la tige d'inhalation comprend un cylindre d'inhalation, un embout d'inhalation, un atomiseur disposé dans le cylindre d'inhalation, et une coupelle de stockage de liquide de cigarette pour stocker un liquide de cigarette, la tige de batterie comprend un cylindre, une batterie installée dans le cylindre, et un boîtier de fond monté sur un fond du cylindre, le dispositif de connexion est disposé dans le cylindre, et le boîtier de fond recouvre le fond du cylindre de manière à sceller le dispositif de connexion dans l'extrémité du cylindre.

7. Cigarette électronique selon la revendication 6, dans laquelle la coupelle de stockage de liquide de cigarette comprend un siège de coupelle et un couvercle de coupelle, les deux sont espacés l'un de l'autre d'un espace prédéterminé et sont montés de façon serrée sur des parois intérieures du cylindre d'inhalation, un tube de guidage et un élément de stockage de liquide sont tous les deux disposés entre le siège de coupelle et le couvercle de coupelle, le tube de guidage est intérieurement creux avec deux extrémités opposées de celui-ci ouvertes sur l'extérieur, et l'atomiseur est maintenu fixe par le tube de guidage, un tube de positionnement est monté sur des parois extérieures du tube de guidage pour empêcher l'atomiseur de se déplacer dans un sens axial du tube de guidage, le tube de positionnement et le tube de guidage sont ajustés par interférence l'un avec l'autre, et le tube de positionnement bute contre l'atomiseur.

8. Cigarette électronique selon la revendication 7, dans laquelle le siège de coupelle a une structure de coupelle cylindrique, un montant de positionnement formé dans le siège de coupelle pour positionner le tube de guidage, et une chambre interne ronde pour recevoir l'élément de stockage de liquide ; le couvercle de coupelle ayant une structure de couvercle sensiblement cylindrique, une cavité interne circulaire formée dans le couvercle de coupelle pour recevoir le tube de guidage, une pluralité d'évents d'aération formés sur une paroi latérale du couvercle de coupelle, une pluralité de nervures formées sur une paroi supérieure du couvercle de coupelle dans la cavité interne circulaire, chacune de la pluralité de nervures ayant une largeur plus petite qu'un diamètre intérieur du tube de guidage, un fond du couvercle de coupelle s'étendant radialement vers l'extérieur pour former une partie de rebord, dans laquelle la partie de rebord est striée et a des stries multiples, les stries ayant des espaces formés entre celles-ci pour permettre un flux du liquide de cigarette jusqu'à la coupelle de stockage de liquide de cigarette, deux extrémités opposées du tube de guidage sont respectivement positionnées par la paroi supérieure du couvercle de coupelle et le montant de positionnement du siège de coupelle, et la partie de rebord est disposée par rapport à la chambre interne ronde du siège de coupelle pour le positionnement de deux extrémités opposées de l'élément de stockage de liquide.

9. Cigarette électronique selon la revendication 6, dans laquelle la tige d'inhalation et la tige de batterie sont respectivement installées avec un élément de connexion mâle et un connecteur femelle de manière à permettre que la tige d'inhalation et la tige de batterie soient branchées l'une avec l'autre, et les éléments de connexion mâle et femelle ont respectivement des éléments d'électrodes d'atomiseur et des éléments d'électrodes de batterie, dans laquelle l'élément d'électrode d'atomiseur et l'élément d'électrode de batterie sont en contact flexible l'un avec l'autre pour réaliser une connexion électrique.

10. Cigarette électronique selon la revendication 9, dans laquelle l'élément de connexion mâle est sensiblement formé comme un recouvrement rond étant intérieurement creux et a des entrées d'air multiples formées radialement en un fond de l'élément de connexion mâle, et une marche de positionnement est formée par extension radialement vers l'extérieur depuis le fond de l'élément de connexion mâle pour buter contre un fond du cylindre d'inhalation, une première chambre de réception est formée sur le fond de l'élément de connexion mâle pour être montée de façon serrée sur la tige de batterie, des fentes d'électrode sont formées sur une partie médiane de l'élément de connexion mâle et pénètrent l'élément de connexion mâle dans un sens axial de celui-ci, et une projection de positionnement est formée radialement sur le fond de l'élément de connexion mâle et s'étend vers l'extérieur dans le sens axial.

11. Cigarette électronique selon la revendication 10, dans laquelle l'élément de connexion femelle est sensiblement formé comme un recouvrement rond et a une paroi latérale, une paroi supérieure, une chambre interne définie par la paroi latérale et la paroi supérieure, une marche de localisation est formée sur la paroi latérale adjacente à la paroi supérieure et s'étend radialement vers l'extérieur depuis l'élément de connexion femelle pour ajustement sur la tige de batterie, une cheville d'insertion est disposée sur la paroi supérieure et s'étend vers l'extérieur dans un sens axial de l'élément de connexion femelle pour être insérée dans la première chambre de réception de l'élément de connexion mâle, la paroi supérieure est formée concave avec une pluralité de rainures de positionnement pour positionner la projection de positionnement de l'élément de connexion mâle, des trous filetés sont formés dans et pénètrent l'élément de connexion femelle dans le sens axial, deux fentes d'électrode s'étendent dans le sens axial pour positionner les éléments d'électrodes de batterie, et deux canaux internes pour l'insertion des éléments d'électrodes d'atomiseur avec lesquels les éléments d'électrodes de batterie sont en contact flexible dans les canaux internes.

12. Cigarette électronique selon la revendication 9, dans laquelle l'atomiseur comprend au moins un élément chauffant, l'élément d'électrode d'atomiseur incluant un premier élément d'électrode d'atomiseur et un deuxième élément d'électrode d'atomiseur qui se connectent électriquement respectivement avec des électrodes positive et négative de l'au moins un élément chauffant, les éléments d'électrodes de batterie incluent un premier élément d'électrode de batterie et un deuxième élément d'électrode de batterie qui se connectent électriquement respectivement avec des électrodes positive et négative de la batterie, et les premier et deuxième éléments d'électrodes d'atomiseur et les premier et deuxième éléments d'électrodes de batterie sont tous constitués de feuilles métalliques conductrices flexibles.

13. Cigarette électronique selon la revendication 12, dans laquelle le premier élément d'électrode d'atomiseur est une feuille métallique conductrice étant flexiblement déformable et est formé avec une plaque de brasage d'un trou de brasage à une extrémité de celui-ci, une plaque de contact flexible étant flexiblement déformable et formée à une autre extrémité du premier élément d'électrode d'atomiseur, et une plaque d'engagement étant flexiblement déformable et formée en un milieu du premier élément d'électrode d'atomiseur pour un engagement avec l'élément de connexion mâle ; et le deuxième élément d'électrode d'atomiseur a une structure semblable à celle du premier élément d'électrode d'atomiseur.

14. Cigarette électronique selon la revendication 12, dans laquelle le premier élément d'électrode de batterie est une feuille métallique conductrice étant flexiblement déformable et est formé avec une plaque de brasage d'un trou de brasage à une extrémité de celui-ci, une plaque de contact flexible étant flexiblement déformable et formée à une autre extrémité du premier élément d'électrode de batterie, et une plaque d'engagement étant flexiblement déformable et formée en un milieu du premier élément d'électrode de batterie pour un engagement avec l'élément de connexion femelle ; et le deuxième élément d'électrode de batterie a une structure semblable à celle du premier élément d'électrode de batterie.

15. Cigarette électronique selon la revendication 12, dans laquelle les premier et deuxième éléments d'électrodes d'atomiseur et les premier et deuxième éléments d'électrodes de batterie sont chacun formés avec une plaque de brasage ayant un trou de brasage à une extrémité de ceux-ci, une plaque de contact flexible étant flexiblement déformable et formée à une autre extrémité de ceux-ci, et une plaque d'engagement étant flexiblement déformable et formée en un milieu pour un engagement avec un élément de connexion mâle ou femelle correspondant, les premier et deuxième éléments d'électrodes d'atomiseur connectent électriquement respectivement les électrodes négatives de l'au moins un élément chauffant par l'intermédiaire des plaques de brasage et des trous de brasage, les premier et deuxième éléments d'électrodes de batterie connectent électriquement respectivement les électrodes positive et négative de la batterie par l'intermédiaire des plaques de brasage et des trous de brasage, et les premier et deuxième éléments d'électrodes d'atomiseur sont en contact flexible avec les premier et deuxième éléments d'électrodes de batterie par l'intermédiaire des plaques de contact flexibles de ceux-ci pour réaliser une connexion électrique.
